# EUROPEAN PATENT APPLICATION

(11) **EP 0 750 903 A1**
(43) Date of publication of application: **02.01.1997**
(21) Application number: 96201739.8
(22) Date of filing: 24.06.1996
(51) Int. Cl.: A61K 7/32

(54) **Deodorant composition**

(30) Priority: 28.06.1995 NL 1000681
(71) Applicant: Coöperatie Cosun U.A., 4700 BH Roosendaal (NL)
(72) Inventor: Vianen, Gerardus Maria, 4707 DZ Roosendaal (NL); Walraven, Bastiaan Willem, 4707 SR Roosendaal (NL); Meyer, Pieter Diederick, 4706 EJ Roosendaal (NL)
(74) Representative: de Bruijn, Leendert C.

(57) **Abstract**

The invention relates to deodorant compositions which are suitable for preventing or combating human body odour. Said deodorant compositions have, as active ingredient, at least one or more sugar-fatty acid esters, the mono-ester content of the sugar-fatty acid esters having to be at least 30%. The fatty acid component of the active ingredient is formed on the basis of one or more optionally unsaturated fatty acids containing 6 - 22 carbon atoms, which fatty acids are optionally branched and may also contain one or more hydroxyl groups. The sugar component of the active ingredient is, for example, sucrose, glucose, fructose, sorbitol or a mixture thereof. The active ingredient indicated above is used in an amount of 0.01 - 15% by weight in the deodorant composition.

## Description

The invention relates to deodorant compositions which curb or prevent the development of odour as a consequence of the conversion of components of perspiration moisture by microorganisms.

Body odour is produced when components of the odourless perspiration moisture is converted by microorganisms. Microorganisms occurring on the skin are in fact capable of forming substances which have an unpleasant odour from the components of perspiration moisture as well as from skin fat and skin cell residues. In addition to the unpleasant odour, these microbially produced substances can also bring about skin irritation.

More particularly, with reference to the microorganisms responsible for the conversion of constituents of perspiration moisture, reference can be made to Leyden J. J. et al., The microbiology of human axilla and its relationship to axillary odor, J. Invest. Derm. 77 (1981), 413 - 416. In this literature reference, it is pointed out that lipophilic diphtheroids belonging to the skin flora, or *Corynebacterium* varieties, are essentially responsible for the unpleasant odour which originates from the armpit. This aspect is shown diagrammatically in Figure 1 of the Leyden J. J. et al. literature reference.

Further indications that *Corynebacterium* varieties play a part in the production of unpleasant body odour is reported in Viollon C. et al., Ciblage de bactéries cutanées, Parfumes Cosmetiques Arômes, No. 116 (1994), 67 - 70 and in Labows, J. N. et al., Perspectives on axillary odor, J. Soc. Cosmet. Chem., 34 (1982), 193 - 202, the lipophilic diphtheroids, or *Corynebacterium* varieties, being indicated as the most important cause of body odour. In addition, it follows from Smith R. F. et al., A medium for the study of the ecology of human cutaneous diphtheroids, J. Gen. Microbiol., 57 (1969), 411 - 417 that Coryne bacteria occur over the entire human body. In view of the above properties relating to the production of unpleasant odours, the presence of said *Corynebacterium* varieties results in the use of agents which combat or mask said undesirable odour.

More particularly, unpleasant body odour can be avoided as follows:
1) the secretion of perspiration moisture can be reduced or even stopped with the aid of astringents, in particular aluminium salts, such as aluminium chlorohydrate. Astringents denaturize the skin proteins and interfere drastically with the thermal balance of, for example, the armpit;
2) the bacteriae flora of the skin can be reduced with the aid of antimicrobial substances. In this connection, only the microorganisms which cause the unpleasant odour need to be eliminated in the ideal case. Often, however, all the skin microflora are damaged in this process. Sometimes, the microorganisms which do not cause any odour are attacked even more severely;
3) the body odour can be masked with the aid of fragrances. However, the mixture of body odour and perfume may also sometimes have an unpleasant smell.

As an example of a use under (2), mention is made of the use of the bactericide 5-chloro-2-(2,4-dichlorophenoxy)phenol, or Irgasan® DP300 (made by CIBA GEIGY); see, for example, "Handbook of Cosmetic Science, 1st edition, Elsevier Advanced Technology, 1993, page 198. Although said commercial product Irgasan® is very effective per se for this purpose, it contains, however, organically bound halogens and can therefore not be regarded as environmentally friendly. Moreover, very toxic dioxins may be formed during the preparation or during the thermal decomposition of Irgasan®.

The object of the invention is to develop novel deodorising compounds which do not have the abovementioned disadvantages associated with Irgasan® DP300 and also have a specific action against odour-causing microorganisms, in particular *Corynebacterium* varieties in the human skin flora such as *Corynebacterium xerosis* and *Corynebacterium minutissimum*. In addition to the specific action against the odour-causing microorganisms, said novel deodorizing compounds should have no or virtually no action against the other microorganisms belonging to the human skin flora, such as, for example, *Micrococcus luteus* and *Staphylococcus epidermis*.

The object indicated above according to the invention is achieved, surprisingly, with the aid of deodorant compositions which contain at least one or more sugar-fatty acid esters as active ingredient.

The abovementioned action of sugar-fatty acid esters as active ingredient against odour-causing microorganisms of the skin flora such as *Corynebacterium* varieties is all the more surprising since it is known from Suzuki, T. et al., Fermentative production of sucrose- and fructose-fatty acid esters, Japan Kokai 50048186, 75.4.30; Chem. Abstr. 83(15) 130026n CA that the *Corynebacterium* genus is capable of forming the corresponding sucrose- or fructose-fatty acid esters on the basis of sucrose or fructose, respectively, as substrate.

The sugar-fatty acid esters to be used as active ingredient in relation to the deodorant compositions according to the invention are prepared on the basis of a sugar, such as sucrose, glucose, fructose or galactose, or also a polyol such as sorbitol, mannitol, lactitol or xylitol, or a mixture thereof, on the one hand, and an optionally saturated fatty acid containing 6 - 22 carbon atoms, on the other hand, which fatty acids may optionally be branched. Moreover, the fatty acids may also contain one or more hydroxyl groups. Examples of suitable fatty acids are, for example, lauric acid, myristic acid, palmitic acid, stearic acid, oleic acid, hydroxystearic acid and isostearic acid. A method of preparing suitable sugar-fatty acid esters is described, inter alia, in the US Patent Specification 4,778,881. They can also be prepared via hydrolysis of such products. A hydrolysis of, for example, sucrose-fatty acid ester results, in particular, in the forming of (an amount of) glucose-fatty acid esters and fructose-fatty acid esters.

The sugar-fatty acid esters of the deodorant composition according to the invention should have a monoester content of at least 30%, advantageously more than 50%. In the case of 30% monoesters, it may be assumed that 40 - 70% diesters and 0 - 30% triesters or higher esters are normally present in the sugar-fatty acid ester product. One or more of, for example, the abovementioned fatty acids, such as the combination laurate/palmitate, laurate/stearate or palmitate/stearate, can be used as fatty acid component of said esters.

The sugar-fatty acid esters are mild and non-irritant to the human skin. Moreover, said esters are nontoxic, odourless and not susceptible, or barely susceptible, to hydrolysis under the conditions of use.

In addition to the sugar-fatty acid esters as active ingredient, other active ingredients and auxiliaries known from the prior art can be used in the deodorant compositions according to the invention. Examples of such substances are, for example astringents (for example salts of metals such as aluminium, iron, chromium, zinc and zirconium), other antimicrobially active ingredients and/or fragrances; see, for example, CTFA Cosmetic Ingredient Handbook, 2nd edition, 1992, pages 541, 542, 553 and 555. The use of such substances is dependent, inter alia, on the form of the deodorant composition. Examples thereof are, for example, sticks, creams, lotions, powders and aerosols; see, for example, Harry's Cosmeticology, 6th edition, 1973, 251 - 276 and Grundlagen und Rezepturen der Kosmetika, Schräder H., 2nd impression, Part 3, pages 465 - 489, and Jungermann E., Cosmetics and Toiletries, 1995, Part 110, No. 2, pages 49 - 56.

The sugar-fatty acid esters can be used in the ready-to-use deodorant compositions, depending on the form of the latter, in a wide range, for example in a range of 0.01 - 15% by weight, advantageously 0.1 - 10% by weight, and more particularly 0.1 - 5% by weight, calculated on the total weight of the deodorant composition. The ratio of the sugar-fatty acid esters with respect to the other compounds with deodorant activity which may also be used in the deodorant composition is normally in the range from 100:1 to 1:100, advantageously 50:1 to 1:50.

If the sugar-fatty acid esters are used in the form of a solution, they are in an aqueous and/or alcoholic medium. The alkanols suitable for forming the alcoholic medium comprise C₂-C₃-alkanols, glycerols, glycols and polyglycols, such as polyethylene glycol, polypropylene glycol and mixtures thereof.

The deodorant compositions according to the invention can be prepared in accordance with methods known from the prior art. For example, the separate components of the deodorant compositions may be mixed with one another, possibly while heating.

Moreover, the invention relates to a method of combatting human body odour or to a method of combatting the *Corynebacterium* varieties which cause body odour and which occur in the human skin flora.

The invention is explained in greater detail by reference to the examples below, which should not be interpreted as restrictive.

The tests reproduced below have been carried out on the basis of four strains which occur in the human armpit flora, viz. *Micrococcus luteus* ATCC 533, *Staphylococcus epidermis* ATCC 155, *Corynebacterium xerosis* ATCC 373 and *Corynebacterium minutissimum* ATCC 23348 (ATCC = American Type Culture Collection, Maryland 20852, USA).

The *Micrococcus luteus* strain has been incubated at 30°C and the other three strains at 37°C. All strains have been tested for purity and then frozen in glycerol in liquid nitrogen. In addition, the strains have been kept on plate (TSBA medium; TSBA = trypton soya broth (medium CM 129 of Oxoid Ltd. + 1% agar), and regularly re-inoculated (at least once a week).

The sucrose-fatty acid esters used in the examples are:
- Sisterna® L70-C :
   70% laurate/30% palmitate; 70% monoester; and
- Sisterna® SP70-C :
   70% stearate/30% palmitate; 70% monoester.

The abovementioned sucrose-fatty acid esters have been used in concentrations of 400-800-1600-3200-6400 mg/l.

The plate method was made use of as test method. In this method, the medium was prepared in sucrose-fatty acid solutions in the above concentrations. The plate, which was provided with a TSBA medium (see above), was then inoculated with 100 µl of culture and incubated at the desired temperature (see above). In this connection, use was made of overnight cultures in TSB (= trypton soya broth) which have been inoculated in undiluted and diluted form.

The inhibition obtained in the test method of the growth of the respective strains is shown with the values 0 (no inhibition) and with 1, 2, 3, 4 or 5 plus signs, where
- +: 0 - 20% inhibition
- ++: 20 - 40% inhibition
- +++: 40 - 60% inhibition
- ++++: 60 - 80% inhibition
- +++++: 80 - 100% inhibition.

A blank determination without sucrose-fatty acid solution on TSBA was used as a control (pos. cont. TSBA; see Tables A and B in Examples I and II).

The results of the tests are shown in Examples I and II below.

### Example I

The inhibiting action of the sugar esters shown in Table A below were carried out in the above manner.

**TABLE A**

| Sugar ester | | S. epidermis | | M. luteus | | C. xerosis | | C.minutissimus | |
|---|---|---|---|---|---|---|---|---|---|
| type: | conc. | o.n. | 1:100 | o.n. | 1:100 | o.n. | 1:100 | o.n. | 1:100 |
| L70 | 400 | 0 | 0 | 0 | 0 | ++++ | not determined | ++++ | ++++ |
| L70 | 800 | 0 | 0 | 0 | 0 | +++++ | not determined | ++++ | +++++ |
| L70 | 1600 | 0 | 0 | 0 | + | +++++ | not determined | ++++ | +++++ |
| L70 | 3200 | 0 | 0 | + | + | +++++ | not determined | ++++ | +++++ |
| L70 | 6400 | 0 | 0 | + | + | +++++ | not determined | +++ | +++++ |
| SP70 | 400 | 0 | 0 | 0 | + | +++ | not determined | +++ | +++++ |
| SP70 | 800 | 0 | 0 | +++ | +++++ | ++++ | not determined | +++ | +++++ |
| SP70 | 1600 | 0 | 0 | ++++ | +++++ | +++++ | not determined | ++++ | +++++ |
| SP70 | 3200 | 0 | 0 | ++++ | +++++ | +++++ | not determined | ++++ | +++++ |
| SP70 | 6400 | 0 | 0 | +++++ | +++++ | +++++ | not determined | +++++ | no determination |
| pos. cont. TSBA | | 0 | 0 | 0 | 0 | 0 | not determined | 0 | 0 |
| Explanatory note: * Tested sugar esters are: L70 and SP70. * Concentrations used are: 400, 800, 1600, 3200 and 6400 mg/l. * Inoculation: overnight (o.n.) culture and 1:100 dilution of said culture * Method: medium is prepared in sugar ester solution and poured onto the plates. * The plates are inoculated with 100 µl of culture and incubated at the desired temperature. | | | | | | | | | |

### Example II

The inhibiting action of the sugar esters shown in Table B below was carried out in the manner described above.

**TABLE B**

| sucrose ester | | *M. luteus* 1:50 | *C.xerosis* 1:50 | C.minutissimum 1:50 |
|---|---|---|---|---|
| type | conc. | | | |
| L70 | 400 | 0 | ++++ | ++++ |
| L70 | 800 | 0 | +++++ | ++++ |
| L70 | 1600 | + | +++++ | ++++ |
| L70 | 3200 | + | +++++ | ++++ |
| L70 | 6400 | + | +++++ | +++ |
| SP70 | 400 | + | +++ | +++ |
| SP70 | 800 | +++++ | ++++ | +++ |
| SP70 | 1600 | +++++ | +++++ | ++++ |
| SP70 | 3200 | +++++ | +++++ | ++++ |
| SP70 | 6400 | +++++ | +++++ | no determination |
| pos. cont. TSBA | | 0 | 0 | 0 |
| Explanatory note: * Tested sugar esters are: L70 and SP70. * Concentrations used are: 400, 800, 1600, 3200 and 6400 mg/l. * Inoculation: 1:50 dilution of an overnight culture * Method: medium is prepared in sugar ester solution and poured onto the plates. The plates are inoculated with 100 µl of culture and incubated at the desired temperature. | | | | |

### Example III

The inhibiting action of the sugar esters shown in Table C below was carried out in the manner described above.

**TABLE C**

| Ester Type | conc (ug/ml) | S.epidermis | M.luteus | C.xerosis | C.minutissimum |
|---|---|---|---|---|---|
| sucrose behenate | 400 | 0 | 0 | +++ | +++++ |
| sucrose oleate | 400 | 0 | +++ | +++++ | +++++ |
| sucrose isostearate | 400 | 0 | + | +++++ | +++++ |
| sucrose hydroxystearate | 400 | 0 | +++ | +++++ | +++++ |
| glucose/fructose | | | | | |
| palmeate/stearate | 400 | 0 | 0 | ++++ | +++++ |
| sorbitol stearate | 400 | 0 | 0 | 0 | +++++ |
| lactitol stearate | 400 | 0 | +++++ | +++++ | +++++ |
| pos. control (without ester) | | 0 | 0 | 0 | 0 |
| Explanatory note: Inoculation: 1:50 dilution of an overnight culture. Method: medium is prepared in sugar ester solution and poured in Petri-dishes. Then 100 µl of the inoculate is put on the plate and incubated at the desired temperature. | | | | | |

### Example IV

In this example, a general formulation of a deodorant composition according to the invention is illustrated.

| | |
|---|---|
| Water | 60-80% |
| Polyol (propylene glycol) | 0-5% |
| Lipids (stearic acid, mineral oil, beeswax) | 5-15% |
| Emulsifiers (polysorbate 40, sorbitan oleate) | 2-5% |
| Antiperspirant (aluminium chlorohydrate) | 0-25% |
| Sugar ester (Sisterna® L70-C) | approx. 1% |
| Perfume oil | approx. 0.5% |

## Claims

1. Deodorant composition suitable for preventing or combating human body odour, characterized in that the deodorant composition contains at least one or more sugar-fatty acid esters as active ingredient.

2. Deodorant composition according to Claim 1, characterized in that the deodorant composition contains one or more sugar-fatty acid esters having a monoester content of at least 30% as active ingredient.

3. Deodorant composition according to Claim 1 or 2, characterized in that the fatty acid component of the active ingredient is formed on the basis of one or more optionally unsaturated fatty acids containing 6 - 22 carbon atoms, which fatty acids are optionally branched.

4. Deodorant composition according to Claim 3, characterized in that the fatty acids also contain one or more hydroxyl groups.

5. Deodorant composition according to one or more of Claims 1 - 4, characterized in that the fatty acid component of the active ingredient is lauric acid, myristic acid, palmitic acid, stearic acid, oleic acid, hydroxystearic acid and/or isostearic acid.

6. Deodorant composition according to one or more of Claims 1 - 5, characterized in that the sugar component of the active ingredient is sucrose, glucose, fructose, galactose, sorbitol, mannitol, lactitol, xylitol or a mixture thereof.

7. Deodorant composition according to one or more of Claims 1 - 6, characterized in that the active ingredient is present in an amount of 0.01 - 15% by weight, calculated on the basis of the total weight of the deodorant composition.

8. Method of combating the *Corynebacterium* varieties which cause body odour and which occur in human skin flora, characterized in that a deodorant composition according to one or more of Claims 1 - 7 is used in this connection.

9. Method of combating human body odour, characterized in that a deodorant composition according to one or more of Claims 1 - 7 is used in this connection.

10. Use of an active ingredient for the preparation of a deodorant composition, suitable for preventing and that the active ingredient is at least one or more sugar-fatty and esters defined according to any of the claims 1-6.
